# EUROPÄISCHE PATENTANMELDUNG

(11) **EP 2 813 247 A1**
(43) Veröffentlichungstag der Anmeldung: **17.12.2014**
(21) Anmeldenummer: 13002999.4
(22) Anmeldetag: 12.06.2013
(51) Int. Cl.: A61L 2/232, A61L 2/238, A01N 25/10, A01N 59/20

(54) **Antimikrobiell ausgerüstetes Polymer mit eingebundenen Partikel und Desinfektionselement auf Basis des Polymers**

(71) Anmelder: Cu Innotech GmbH, 63477 Maintal (DE); theranovis GmbH & Co. KG, 55411 Bingen (DE)
(72) Erfinder: Schneider, Andreas Dr., 66121 Saarbrücken (DE); Zeiger, Marco, 66583 Elversberg (DE); Frontzek, Christian Amadeus, 63450 Hanau (DE)
(74) Vertreter: Cornelius, Thomas

(57) **Zusammenfassung**

Ein Desinfektionselement (1,1') mit einem mikrobizid ausgebildeten Berührungsbereich (2,2') auf Kupferbasis soll eine besonders hohe Vielfalt an möglichen Anwendungen bei zuverlässig hoher mikrobizider Wirksamkeit in seinem Berührungsbereich (2,2') ermöglichen. Dazu weist der Berührungsbereich (2,2') erfindungsgemäß eine mikrobizide, aus in eine Matrix (8) eingebetteten Kupfer-Partikeln (10) gebildete Oberflächenschicht (6) auf.

## Beschreibung

Die Erfindung betrifft ein Desinfektionselement mit einem mikrobizid ausgebildeten Berührungsbereich. Sie bezieht sich weiter auf ein Verfahren zur Herstellung eines derartigen Desinfektionselements.

Im Alltag kommen Menschen häufig unbewusst mit gesundheitsschädlichen Bakterien, Keimen und Viren in Berührung. Besonders dort, wo man mit Flächen in Kontakt kommt, die von vielen anderen Menschen zuvor berührt worden sind, kann eine zunehmende Ansammlung von Keimen vermehrt zu unhygienischen Bedingungen führen. Insbesondere in öffentlichen Institutionen und Verkehrsmitteln kommt man mit Gegenständen, wie zum Beispiel Türgriffen und anderen Berührungsflächen in Kontakt, auf denen sich Bakterien ansammeln und vermehren können. Selbst in Institutionen, die eigentlich strenge Hygienevorschriften befolgen, wie beispielsweise in Krankenhäusern und Kindergärten, können Krankheitserreger übertragen werden und so die Gesundheit schädigen.

Um dem entgegenzuwirken, können derartige Gegenstände mikrobizid in dem Sinne ausgerüstet werden, dass Oberflächen oder Oberflächenbereiche keim- oder bakterientötend ausgeführt sind. Zu diesem Zweck kann ein Desinfektionselement mit einem entsprechend mikrobizid oder keimtötend ausgeführten Berührungsbereich vorgesehen sein. Einem derartigen Desinfektionselement liegt die Auslegung zugrunde, dass in dem zur Berührung vorgesehenen Berührungsbereich, beispielsweise der Oberfläche eines Griffs, einer Drückertaste oder dergleichen, die äußere oder freiliegende und damit der Berührung ausgesetzte Oberfläche mit einem geeignet gewählten mikrobiziden Material versehen wird, so dass die sich in Folge der wiederkehrenden Berührungen ansammelnden Keime oder Mikroorganismen unmittelbar wieder abgetötet werden und sich damit bereits von vornherein nicht vollständig ansiedeln können.

Ein derartiges Desinfektionselement mit einem mikrobizid ausgeführten Berührungsbereich ist beispielsweise aus der DE 10 2009 013 029 A1 bekannt.

Dieses bekannte Desinfektionselement basiert von seiner Auslegung her auf der Nutzung von Kupfer als Basismaterial für die angestrebte mikrobizide Wirkung.

Diese Nutzung von Kupfer wird auch vorliegend als besonders vorteilhaft angesehen, da gerade Kupfer eine Vielzahl von für diesen Einsatzzweck besonders günstigen Eigenschaften aufweist. Einerseits zeigt nämlich Kupfer als Material für viele Mikroorganismen schon in geringen Mengen eine toxische Wirkung und kann somit besonders zuverlässig zur Abtötung von Keimen oder Mikroorganismen verwendet werden. Andererseits ist Kupfer aber - im Gegensatz beispielsweise zu Silber oder anderen mikrobiziden Grundstoffen - Bestandteil des natürlichen menschlichen Stoffwechsels und daher grundsätzlich für den menschlichen Organismus unschädlich.

Eine Kontamination des menschlichen Organismus durch ein Desinfektionselement auf Kupferbasis ist daher selbst bei hoher mikrobizider Wirkung nicht zu befürchten. Die nachfolgenden Ausführungen beziehen sich daher auf die als besonders vorteilhaft angesehene Verwendung von Kupfer als mikrobizider Basisstoff. In analoger und ebenfalls als im Sinne der vorliegenden Erfindung angesehene Ausgestaltung kann in denjenigen Fällen, bei denen eine besonders gute Verträglichkeit mit dem menschlichen Organismus kein Auslegungsziel mit hoher Priorität darstellt, anstelle von Kupfer grundsätzlich auch ein anderer geeigneter mikrobizider Grundstoff wie beispielsweise Silber, Zink oder dergleichen oder auch eine Mischung dieser Materialien vorgesehen sein.

Die genannten Eigenschaften von Kupfer als mikrobizider Basisstoff werden beim Desinfektionselement nach der DE 10 2009 013 029 A1 gezielt genutzt. Bei diesem bekannten Desinfektionselement ist zudem gezielt dem Umstand Rechnung getragen, dass für eine ausreichend hohe mikrobizide oder keimtötende Wirkung ein Mindestmaß der Anzahl der pro Flächen- und Zeiteinheit an die mit dem Berührungsbereich in Kontakt tretenden Gewebeflächen abgegebenen Kupferionen, nachfolgend auch als "Ionenausschüttung" bezeichnet, eingehalten werden sollte.

Das aus der DE 10 2009 013 029 A1 bekannte Desinfektionselement ist in diesem Sinne für eine besonders hohe lonenauschüttung im Berührungsbereich ausgelegt und umfasst zu diesem Zweck im Berührungsbereich eine Oberflächenstruktur auf Kupferbasis, die eine im Vergleich zur äußeren Oberfläche vergrößerte innere Oberfläche aufweist.

Diese Auslegung des bekannten Desinfektionselements setzt allerdings eine vergleichsweise massive Bauweise des Berührungsbereichs in seiner frei liegenden Oberfläche voraus. Das bekannte Desinfektionselement ist daher in seinen möglichen Anwendungsbereichen nur begrenzt flexibel.

Der Erfindung liegt daher die Aufgabe zugrunde, ein Desinfektionselement der oben genannten Art anzugeben, das eine besonders hohe Vielfalt an möglichen Anwendungen bei zuverlässig hoher mikrobizider Wirksamkeit in seinem Berührungsbereich ermöglicht. Des Weiteren soll ein zur Herstellung eines derartigen Desinfektionselements besonders geeignetes Verfahren angegeben werden.

Bezüglich des Desinfektionselements wird diese Aufgabe erfindungsgemäß gelöst, indem der Berührungsbereich eine mikrobizide, aus in eine Matrix eingebetteten Kupfer-Partikeln gebildete Oberflächenschicht aufweist.

Die Erfindung geht von der Überlegung aus, dass im Hinblick auf die genannten besonders günstigen Eigenschaften von Kupfer für den vorgesehenen Einsatzzweck dieses unverändert als Basisstoff für die mikrobizide Ausrüstung des Berührungsbereichs vorgesehen sein sollte. Um aber dennoch eine besonders hohe Flexibilität beim Einsatz und der Ausgestaltung des Berührungsbereichs zu ermöglichen, sollte vom Einsatz eines massiven Kupferblocks abgesehen werden.

Stattdessen ist vorgesehen, das für die Bereitstellung der mikrobiziden Eigenschaften vorgesehene Kupfer in eine Matrix aus einem geeignet gewählten Trägermaterial einzubinden. Dieses sollte insbesondere zunächst in flüssiger, später aushärtbarer Form vorliegen, so dass das mit Kupferpartikeln versehene Matrixmaterial auf besonders einfache Weise einer Formgebung unterzogen werden kann. Auf diese Weise ist eine zuverlässige Ausrüstung auch vergleichsweise komplexer Geometrien mit einer mikrobiziden Oberflächenschicht und damit eine besonders große Vielzahl an möglichen räumlichen Ausgestaltungen des Desinfektionselements oder von dessen Berührungsbereich möglich.

Das Desinfektionselement kann dabei in der Art eines Festkörpers als Vollkörper ausgeführt sein, der vollständig von dem ausgehärteten, mit den eingebetteten Kupfer-Partikeln versehenen Matrixmaterial gebildet wird.

Dabei kann das Desinfektionselement beispielsweise in der Art eines Gusskörpers hergestellt sein, wobei bei der Herstellung auf herkömmliche, für das jeweils vorgesehene Matrixmaterial geeignet gewählte Verfahren zurückgegriffen werden kann. Dabei kann das solchermaßen gebildete Desinfektionselement mit einer zumindest annähernd räumlich homogenen, gleichmäßigen Verteilung der eingebetteten Kupfer-Partikel versehen sein, bei der sämtliche Raumbereiche des Desinfektionselements eine vergleichbare Konzentration an eingebetteten Kupfer-Partikeln aufweisen. In besonders vorteilhafter Ausgestaltung kann die Konzentration der eingebetteten Kupfer-Partikel aber auch räumlich inhomogen und im Oberflächen-nahen Bereich überhöht sein, so dass der überwiegende Anteil der Kupfer-Partikel nah zur Oberfläche des Berührungsbereichs positioniert ist.

Ein derartiger, gezielt gewählter Konzentrationsverlauf der Kupfer-Partikel ist beispielsweise erhältlich, indem die Kupfer-Partikel einem zur Bildung der Matrix vorgesehenen Ausgangsmaterial beigemischt und in diesem vorübergehend in Suspension oder Schwebe gehalten werden. Während des Aushärtens des Ausgangsmaterials zur Bildung der Matrix können die Kupfer-Partikel schwerkraftbedingt zu Boden sinken, so dass eine Anreicherung der Partikel im unteren Grenzflächenbereich des entstehenden Gusskörpers erfolgt. Dieser untere Grenzflächenbereich kann sodann zur Bildung des Berührungsbereichs herangezogen werden.

Die Verteilung der Partikel im Gusskörper kann dabei durch geeignete Parameterwahl bei der Herstellung besonders bedarfsgerecht eingestellt werden. Insbesondere durch eine geeignete Wahl der Prozesszeiten, also insbesondere der für die Aufkonzentration der Partikel im Grenzflächenbereich gewählten Zeit, im Hinblick auf die Stoffeigenschaften des zur Bildung der Matrix vorgesehenen Ausgangsmaterials, insbesondere dessen Viskosität, kann dabei eine gewünschte Konzentration der Partikel im oberflächennahen Bereich zuverlässig und reproduzierbar eingestellt werden.

In alternativer vorteilhafter Ausgestaltung ist die mikrobizide Oberflächenschicht des Berührungsbereichs als auf einen Tragkörper aufgebrachte Beschichtung ausgeführt. Damit ist eine besonders weitgehende Flexibilität insbesondere hinsichtlich möglicher Geometrieformen erreichbar. Die Einbindung der Kupferpartikel in das Matrixmaterial ermöglicht dabei eine besonders flexible Anwendung des mikrobiziden Materials auch an verschiedenartig geformten oder ausgestalteten Flächenbereichen des Tragkörpers.

Bei der Einbettung der Kupferpartikel in das Matrixmaterial wird vorteilhafterweise in besonderem Maße berücksichtigt, dass zur frei liegenden Außenoberfläche des Berührungsbereichs hin eine ausreichend hohe Ionenausschüttung an Kupferionen stattfindet, so dass die mikrobizide Wirkung zuverlässig aufrechterhalten werden kann. Dies wird einerseits durch die oben genannte Aufkonzentration von Kupfer-Partikeln in Oberflächennähe begünstigt. Wie sich überraschenderweise herausgestellt hat, ist dies andererseits aber auch auf besonders günstige Weise erreichbar, indem in vorteilhafter Ausgestaltung die Kupferpartikel in der Matrix als so genannte Nano- oder Mikropartikel, also als Partikel mit einer durchschnittlichen Größe im Nano- oder Mikrometerbereich, vorliegen.

In besonders vorteilhafter Ausgestaltung umfassen somit die in die Matrix eingebetteten Kupfer-Partikel eine Partikelfraktion mit einer Partikelgröße von höchstens 500 nm, besonders bevorzugt mit einer Partikelgröße von 90 bis 250 nm, und/oder eine Partikelfraktion mit einer Partikelgröße von mindestens 1 µm. Die Partikelfraktion weist dabei besonders bevorzugt eine mittlere Partikelgröße von etwa 20 µm bis 50 µm und/oder eine Standardabweichung von höchstens 20% auf. Durch eine derartige Wahl der Partikelgröße im Nano- oder Mikrometerbereich ist unter anderem sichergestellt, dass die Partikel ein besonders großes Oberflächen-Volumen-Verhältnis und damit eine besonders große spezifische Oberfläche aufweisen, was die Ionenabgabe sehr begünstigt.

Im Hinblick auf eine besonders günstige Verarbeitbarkeit und Applizierbarkeit des vorgesehenen kupferhaltigen Mikrobizids wird das zur Bildung der Matrix vorgesehene Material zweckmäßigerweise gezielt ausgewählt. Dabei sollte als Auslegungsparameter unter anderem einerseits die Fähigkeit berücksichtigt werden, die Kupferpartikel zuverlässig und gff. auch gleichmäßig zu dispergieren, so dass möglichst keine oder nur geringe Agglomeration stattfindet.

Andererseits sollte aber auch eine besonders gute Verarbeitbarkeit bei der Formgebung, also bei der Herstellung eines Gusskörpers und/oder bei der Aufbringung der Beschichtung, angestrebt werden. Dazu ist insbesondere das Vorliegen eines Ausgangsmaterials als Flüssigkeit, in die die Kupferpartikel eingebracht werden können und die anschließend nach der Formgebung oder Beschichtung ausgehärtet werden kann, wünschenswert. Im Hinblick auf diese Kriterien ist die Matrix vorteilhafterweise aus Polymermaterial, vorzugsweise aus organischen oder anorganischen Strukturelementen, aus (Poly) Paraphynelen, aus einem Epoxyharz und/oder aus Polyurethan gebildet.

Die auslegungsbedingt vorgesehene und auch erwünschte ausreichend hohe lonenausschüttung oder Ionenabgabe könnte einerseits dadurch sichergestellt werden, dass als Matrixmaterial vorteilhafterweise ein Material gewählt wird, das gerade für Kupferionen eine besonders hohe intrinsische Durchlässigkeit aufweist und somit eine lonenwanderung von Kupferionen möglichst nur geringfügig behindert. Alternativ oder zusätzlich wird die Ionenabgabe aber auch dadurch begünstigt, dass in besonders vorteilhafter Ausgestaltung der Berührungsbereich des Desinfektionselements derart ausgeführt ist, dass die Kupfer-Partikel im Oberflächenbereich der Beschichtung zumindest teilweise unbedeckt vom Matrixmaterial vorliegen, so dass gegebenenfalls ein direkter mechanischer Kontakt der Partikel mit sich ansiedelnden Keimen ermöglicht ist.

Wie sich nämlich völlig überraschend herausgestellt hat, ist neben der Ionenabgabe der direkte Kontakt der Keime mit Kupfer oder den Kupferpartikeln besonders günstig für die antimikrobielle Aktivität. Gerade durch die oben genannten Partikelgrößen ist ein als besonders günstig angesehener Kompromiß zwischen hoher Ionenabgabe und größtmöglicher fei liegender Kontaktfläche an der Oberfläche des Desinfektionselemts erreichbar. Es wird vermutet, dass offene oder freiliegende Kupferflächen an der Kontaktoberfläche eine Schädigung der Zellhülle der Mikroben bewirken und so das Eindringen der Kupferionen in das Zellinnere besonders begünstigen; demzufolge kann in alternativer besonders vorteilhafter Ausgestaltung auch ein anderes Material als Kontaktmaterial für die Mikroben gewählt werden, das ebenfalls eine derartige gezielte Schwächung der Zellhülle bewirkt.

Die Bereitstellung derartiger unbedeckter oder frei liegender Kupferflächen kann bei geeigneter Wahl der Dimensionierungsparameter (insbesondere Partikelgröße in Kombination mit der Dicke der Beschichtung) erreicht werden, indem die Kupfer-Partikel vorteilhafterweise zumindest teilweise nach oben hin unbedeckt aus dem Matrixmaterial herausragen.

In alternativer oder zusätzlicher vorteilhafter Ausgestaltung kann auch ein Aufrauhungs- und/oder Ätzprozeß der Matrix vorgesehen sein, mit dem das Matrixmaterial oberhalb der eingebundenen Kupfer-Partikel zumindest teilweise entfernt und deren Oberfläche somit zumindest teilweise freigelegt wird. Dies erfolgt besonders bevorzugt durch ein mechanisches Verfahren, beispielsweise Schleifen. Die mikrobizide Wirkung eines derartig hergestellten Materialverbundes mit frei liegendem Kupferanteil an der Oberfläche hat insbesondere den Vorteil, dass insofern keinerlei Behinderung der Ionenabgabe oder lonenausschüttung oder des direkten Kontakts von Zellwand und Kupferfläche durch das Matrixmaterial vorliegt.

Für die gewünschte ausreichend hohe Ionenausschüttung und/oder den frei liegenden Kontaktbereich ist dabei besonders vorteilhaft vorgesehen, dass der durch die frei liegenden Kupfer-Partikel gebildete Anteil der Gesamtoberfläche der Beschichtung mindestens 10 % beträgt.

Vorteilhafterweise ist bei der Ausgestaltung des Desinfektionselements zusätzlich zu dessen Wirksamkeit (erreichbar durch eine ausreichend hohe Ionenauschüttung oder - abgabe und/oder durch möglichst und weitgehend direkten mechanischen Kontakt des partiell eingebetteten Kupfers zu den sich ansiedelnden Keimen) auch noch dessen mechanische Belastbarkeit und damit Langzeitstabilität selbst bei vorliegendem Abrieb oder sonstiger mechanischer Beanspruchung besonders berücksichtigt. Im Sinne einer ausreichend hohen mechanischen Stabilität sollte das Matrixmaterial, das in der Art eines Bindemittels die mechanische Stabilität unter den Kupfer-Partikeln herstellt, einen geeignet gewählten Volumenanteil an der Beschichtung insgesamt einnehmen. Um dem Rechnung zu tragen, beträgt vorteilhafterweise der Volumenanteil der Kupfer-Partikel am Gesamtvolumen der Beschichtung zwischen 10% und 80%.

Zweckmäßigerweise weist die den Berührungsbereich des Desinfektionselements bildende Oberflächenschicht eine Schichtdicke von mindestens 1 µm, insbesondere von mindestens 10 µm, und von höchstens 100 µm auf. Diese Abmessungen sind besonders günstig, da man einerseits eine gewisse Mindestanzahl an Kupfer-Partikeln für die Abtötung der Mikroorganismen oder Keime benötigt.

Andererseits sollte die Dicke aber gerade im Hinblick auf die vorgesehene Teilchengröße der Kupfer-Mikropartikel nicht zu groß gewählt sein, damit auf besonders einfache Weise zumindest ein Teil der Partikeloberfläche vom Matrixmaterial unbedeckt gehalten werden kann.

Bezüglich des Herstellungsverfahrens für das Desinfektionselement wird die genannte Aufgabe gelöst, indem Kupfer-Partikel in eine Lösung eines Matrixbildners, vorzugsweise auf Polymerbasis, eingerührt werden und die dadurch erhaltene Suspension oder Dispersion anschließend homogenisiert wird. Dieses Ausgangsmaterial kann sodann einer Formgebung unterzogen und anschließend ausgehärtet werden, so dass ein zur Bildung des Desinfektionselements oder von dessen Berührungsbereich vorgesehener Gusskörper entsteht. Alternativ kann ein Trägerkörper zumindest teilweise mit einer mit den Kupfer-Partikeln und dem Matrixbildner versehenen Beschichtungslösung beschichtet werden. In ganz besonders vorteilhafter Ausgestaltung ist zu dieser Herstellung des Desinfektionselements ein Beschichtungsprozeß durch Abscheidung einer mit Kupfer-Partikeln und einem Matrixbildner versehenen Beschichtungslösung auf einem Trägerkörper durch Tauchbeschichtung zur Bildung des Berührungsbereichs vorgesehen.

Um die Wirksamkeit der hergestellten Beschichtung zuverlässig sicherzustellen und insbesondere eine ausreichend hohe Ionenausschüttung, ggf. verbunden mit einer großen Kontaktfläche, zu ermöglichen, werden die Kupfer-Partikel vorteilhafterweise vor dem Einbringen in die Lösung des Matrixbildners stabilisiert, so dass Agglomeration der Partikel weitgehend vermieden wird und eine besonders große äußere Oberfläche der Kupfer-Partikel-Fraktion entsteht. Dabei werden die Partikel vorzugsweise durch eine Ultraschallbehandlung in einer Flüssigkeit, vorzugsweise Ethanol, dispergiert, so dass sie weitgehend als einzelne Partikel getrennt vorliegen.

Danach wird die Oberfläche der Kupfer-Partikel durch eine geeignet gewählte chemische Komponente, bevorzugt Polyvinylpyrrolidon (PVP), funktionalisiert, so dass ein Verklumpen oder eine Agglomeration verhindert wird (so genannte sterische Stabilisierung). Um eine dünne Schicht auf dem Tragkörper zu erreichen, kann diese in einer vorteilhaften Variante beispielsweise mit einer Dispersionsbeschichtung aufgebracht werden, wobei das im Beschichtungs-Verfahren verwendete Material als eine Dispersion in einem Lösungsmittel fein verteilt ist.

Die Dispersion wird mittels Pressluft zu einem Nebel zerstäubt und gleichmäßig auf das Substrat gesprüht. Das Substrat wird anschließend in einem Ofen erhitzt, so dass sich eine dünne Schicht ausbildet.

Die mit der Erfindung erzielten Vorteile bestehen insbesondere darin, dass durch die Ausrüstung des Matrix-Materials, insbesondere eines Polymers, mit eingebetteten Kupfer-Partikeln ein vergleichsweise gut und flexibel verarbeitbares Material bereitgestellt werden kann, das eine antimikrobielle oder mikrobizide Wirkung aufweist und somit besonders günstig und zuverlässig zu Desinfektionszwecken genutzt werden kann. Die besonders hohe Flexibilität gerade bei der Endverarbeitung durch geeignete Formgebung oder auch durch Aufbringen in Form einer Beschichtung ermöglicht dabei eine große Vielzahl an denkbaren Anwendungsmöglichkeiten und -gebieten.

Durch die Einbettung der Kupfer-Partikel, insbesondere Kupfer-Nano- oder Mikropartikel, in ein Matrixmaterial kann eine hohe Kupfer-Ionenausschüttung auch für Berührungsbereiche mit vergleichsweise komplexer Geometrie oder auch in großer Stückzahl sichergestellt werden. Damit ist die zuverlässige Bereitstellung eines mikrobiziden Berührungsbereichs auch bei flexiblen oder variierenden Anforderungen auf besonders einfache Weise ermöglicht.

Ein Ausführungsbeispiel der Erfindung wird anhand einer Zeichnung näher erläutert. Darin zeigen
- Fig. 1 - 4: jeweils ein Desinfektionselement in Schnittdarstellung,
- Fig. 5 - 7: jeweils Diagramme zum zeitlichen Verlauf der Kupfer-Ionenabgabe, und
- Fig. 8 - 10: jeweils Diagramme zum zeitlichen Verlauf der Bakterienanzahl.

Gleiche Teile sind in allen Figuren mit denselben Bezugszeichen versehen.

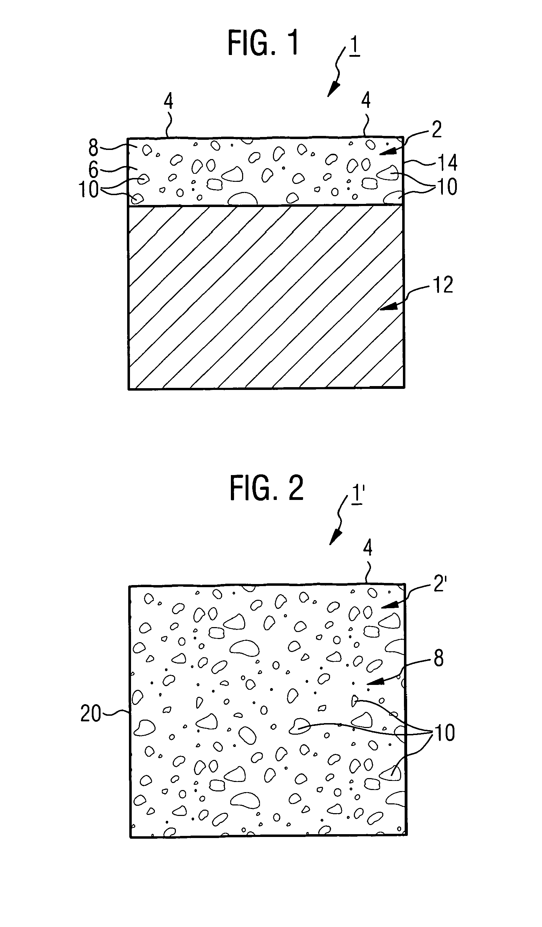

Das Desinfektionselement 1 gem. Fig. 1 ist zur Verwendung an oder in Gegenständen oder allgemein Oberflächen vorgesehen, bei denen mit häufigen Berührungen durch eine Vielzahl von Personen zu rechnen ist, wie beispielsweise Türgriffen oder Geländern in öffentlichen Gebäuden, oder bei denen aus sonstigen Gründen auf ein besonders hohes Maß an Keimfreiheit oder Sterilität zu achten ist, wie beispielsweise in Krankenhäusern. Um dabei einer Verkeimung oder Verunreinigung durch Mikroorganismen gezielt und konsequent entgegenzuwirken, ist das Desinfektionselement 1 in seinem für die Berührung durch Personen vorgesehenen Berührungsbereich 2 mikrobizid oder keimtötend ausgeführt. Der Berührungsbereich 2 ist dabei in seinem Oberflächenbereich unter Verwendung von Kupfer als mikrobizides Basismaterial ausgeführt, um die für diesen Zweck äußerst günstigen Eigenschaften von Kupfer gerzielt nutzen zu können. Insbesondere soll dabei gezielt genutzt werden, dass Kupfer einerseits für viele Mikroorganismen schon in geringen Mengen eine toxische Wirkung zeigt, so dass es besonders zuverlässig zur Abtötung von Keimen oder Mikroorganismen verwendet werden kann, wobei es andererseits aber - im Gegensatz beispielsweise zu Silber oder anderen mikrobiziden Grundstoffen - Bestandteil des natürlichen menschlichen Stoffwechsels und daher grundsätzlich für den menschlichen Organismus unschädlich ist.

Das Desinfektionselement 1 ist für eine besonders hohe Flexibilität und variable Einsatzmöglichkeiten ausgeführt. Um dies zu ermöglichen, weist der Berührungsbereich 2 an seiner frei zugänglichen und somit berührbaren Oberfläche 4 eine mikrobizide Oberflächenschicht 6 auf. Diese mikrobizide Oberflächenschicht 6 wird durch eine Matrix 8 gebildet, in die als eigentlich mikrobizides Material Kupfer-Partikel 10 eingebettet sind.

Im Hinblick auf die gewünschte Wirkungsweise, also der gezielten Nutzung der mikrobiziden Eigenschaften des in die Matrix 8 eingelagerten Kupfers, ist die Oberflächenschicht 6 gezielt auf eine ausreichend hohe Ausschüttung von Kupferionen an den Oberflächenbereich ausgeführt. Wie sich überraschenderweise herausgestellt hat, ist dies auf besonders günstige Weise erreichbar, indem die Kupferpartikel 10 in der Matrix 8 als so genannte Nano- oder Mikropartikel, also als Partikel mit einer durchschnittlichen Größe im Nano- bzw. Mikrometerbereich, vorliegen.

Dementsprechend weisen die in die Matrix 8 eingebetteten Kupfer-Partikel 10 im Ausführungsbeispiel eine mittlere Partikelgröße von etwa 20 - 50 µm, mit einer Standardabweichung von höchstens 20%, auf. In alternativer vorteilhafter Ausgestaltung können die Kupfer-Partikel 10 auch eine Partikelfraktion mit einer Partikelgröße von höchstens 500 nm, vorzugsweise mit einer Partikelgröße von 90 bis 250 nm, umfassen.

Die Kupfer-Partikel 10 können in sich weitgehend homogen und als Vollkörper ausgeführt sein und insbesondere aus reinem oder nahezu reinem Kupfer, ggf. auch aus einer geeignet gewählten Legierung bestehen. Alternativ und bevorzugt ist aber auch ein besonders materialsparender Einsatz des Kupfers denkbar, indem die Kupfer-Partikel 10 ihrerseits als außenseitig mit Kupfer beschichtete Partikel eines Trägermaterials ausgeführt sind. Diese Partikel können beispielsweise außenseitig unter Rückgriff auf die Wirbelschichttechnik mit einer Hülle aus Kupfer beschichtet werden.

Die Wirbelschichttechnik ermöglicht, ähnlich einer Plasmabeschichtung, jedoch speziell für Kleinstpartikel, im industriellen Maßstab zu beschichten. Für die spezielle Anwendung, also insbesondere die Beschichtung mit Kupfer, besonders geeignet und daher besonders bevorzugt sind als Basis- oder Trägermaterial insbesondere Blähton oder auch vergleichsweise leichte Metalle und/oder Metalle, die selbst eine antimikrobielle Wirkung haben. Durch eine derartige Ausgestaltung der Partikel 10 kann zusätzlich zu einem besonders wirksamen Ressourceneinsatz und der dadurch erreichbaren Kostensenkung auch eine verbesserte Oberflächenbeschaffenheit im Endprodukt und/oder auch eine Optimierung des Wirkmechanismus erreicht werden.

Hinsichtlich der Ausgestaltung der Oberflächenschicht 6 sind mehrere als besonders vorteilhaft angesehene Varianten denkbar. Im Ausführungsbeispiel gem. FIG. 1 ist die Oberflächenschicht 6 als auf einen Tragkörper12 aufgebrachte Beschichtung 14 ausgeführt.

Das zur Bildung der Matrix 8 vorgesehene Material ist gezielt im Hinblick auf eine günstige Verarbeitbarkeit und Applizierbarkeit ausgewählt. Insbesondere ist zur Herstellung des Berührungsbereichs 2 bzw. von dessen Oberflächenschicht 6 nämlich vorgesehen, die die Kupfer-Partikel 10 enthaltende Matrix 8 durch ein Tauchbeschichtungsverfahren aufzubringen.

Dementsprechend ist das zur Bildung der Matrix 8 vorgesehene Ausgangsmaterial gezielt unter Berücksichtigung der Auslegungsparameter gewählt, dass unter anderem einerseits die Kupferpartikel 10 zuverlässig und gff. auch gleichmäßig aufgenommen werden, und dass andererseits eine besonders gute Verarbeitbarkeit zur Aufbringung der Beschichtung 14 gegeben ist. Dabei ist vorgesehen, die Kupferpartikel 10 nach geeigneter Vorbereitung, insbesondere nach einem Stabilisierungsschritt, in eine Flüssigkeit einzubringen und in dieser zu homogenisieren und gleichmäßig zu verteilen. Anschließend soll die mit den Partikeln 10 beladene Flüssigkeit im Rahmen einer Tauchbeschichtung auf den Tragkörper 12 aufgebracht werden, und anschließend ist ein Aushärteschritt, ggf. mit Unterstützung durch thermische Behandlung, zur Bildung der eigentlichen Matrix 8 vorgesehen. Im Hinblick auf diese Kriterien ist als das zur Bildung der Matrix 8 vorgesehene Ausgangsmaterial ein Polymermaterial, vorzugsweise aus organischen oder anorganischen Strukturelementen, Paraphynelen, ein Epoxyharz und/oder Polyurethan gewählt.

Im alternativen Ausführungsbeispiel gem. FIG. 2 ist das Desinfektionselement 1' hingegen zumindest in seinem Berührungsbereich 2' als Guß- oder Vollkörper 20 ausgeführt.

Dieser wird durch das ausgehärtete, mit den eingebetteten Kupfer-Partikeln 10 versehene Matrixmaterial gebildet. Im Hinblick auch auf diese Kriterien ist als das zur Bildung der Matrix 8 vorgesehene Ausgangsmaterial ebenfalls ein Polymermaterial, vorzugsweise aus organischen oder anorganischen Strukturelementen, Paraphynelen, ein Epoxyharz und/oder Polyurethan gewählt; diese Materialien sind somit als Matrixbildner für beide Varianten, also zur Herstellung einer Beschichtung 14 ebenso wie zur Herstellung eines Guß- oder Vollkörpers, gleichermaßen geeignet und bevorzugt. Bei der Herstellung des Vollkörpers 20 kann auf herkömmliche, auf das verwendete Matrixmaterial geeignet abgestimmte Verfahren zurückgegriffen werden.

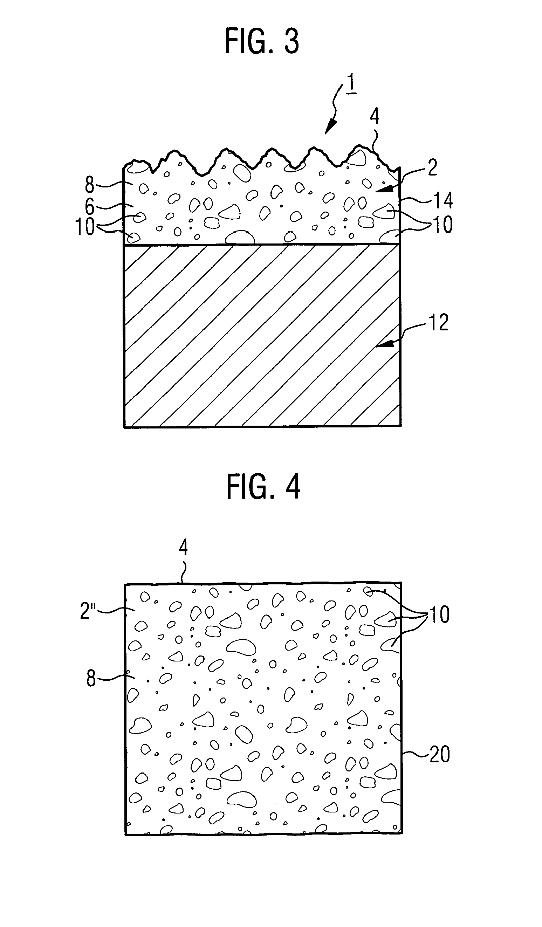

In den in FIG. 1,2 gezeigten Ausführungsbeispielen sind die Kupfer-Partikel 10 im Wesentlichen vollständig in die Matrix 8 eingebettet und von dieser umschlossen. Um die auslegungsbedingt vorgesehene und auch erwünschte ausreichend hohe lonenausschüttung oder - abgabe noch weiter zu begünstigen, kann der Berührungsbereich 2 des Desinfektionselements 1 aber in einer besonders bevorzugten, in FIG. 3 dargestellten Ausführungsform auch derart ausgestaltet sein, dass die Kupfer-Partikel 10 im Oberflächenbereich der Oberflächenschicht 6 zumindest teilweise unbedeckt von der Matrix 8 vorliegen. Damit ist zusätzlich zu einer insgesamt erhöhten Ionenausschüttung im frei liegenden Berich der Partikel ein dirketer Kontakt von (Keim-) Zellhüllen mit Kupferatomen möglich, so dass gezielt die Zellhüllen geschwächt oder geschädigt werden können. Zur Herstellung der entsprechenden Oberflächenbereiche ist vorzugsweise ein Aufrauhungs- und/oder Ätzprozeß der Matrix 8 vorgesehen, mit dem das Matrixmaterial oberhalb der eingebundenen Kupfer-Partikel 10 zumindest teilweise entfernt und deren Oberfläche somit zumindest teilweise freigelegt wird. Als besonders zuverlässig und somit besonders bevorzugt hat sich zu diesem Zweck eine Anrauung der Oberfläche durch mechanisches Schleifen erwiesen.

Im Ausführungsbeispiel und besonders bevorzugt beträgt der durch die frei liegenden Kupfer-Partikel 10 gebildete Anteil der Gesamtoberfläche der Oberflächenschicht 6 mindestens 10%.

In den Ausführungsbeispielen gem. FIG. 1 bis 3 ist die Oberflächenschicht 6 mit einer zumindest annähernd räumlich homogenen, gleichmäßigen Verteilung der eingebetteten Kupfer-Partikel 10 ausgeführt, bei der sämtliche Raumbereiche der Oberflächenschicht 6 eine vergleichbare Konzentration an eingebetteten Kupfer-Partikeln 10 aufweisen.

In dem alternativen, ebenfalls als besonders vorteilhaft angesehenen Ausführungsbeispiel gem. FIG. 4 ist die Konzentration der eingebetteten Kupfer-Partikel 10 aber hingegen räumlich inhomogen, wobei im Bereich nahe der Oberfläche 4 eine Überhöhung vorliegt, so dass der überwiegende Anteil der Kupfer-Partikel 10 nah zur Oberfläche 4 des Berührungsbereichs 2" positioniert ist. Ein derartiger, gezielt gewählter Konzentrationsverlauf der Kupfer-Partikel 10 ist beispielsweise erhältlich, indem die Kupfer-Partikel 10 dem zur Bildung der Matrix 8 vorgesehenen Ausgangsmaterial beigemischt und in diesem vorübergehend in Suspension oder Schwebe gehalten werden. Während des Aushärtens des Ausgangsmaterials zur Bildung der Matrix 8 können die Kupfer-Partikel 10 schwerkraftbedingt zu Boden sinken, so dass eine Anreicherung der Partikel im unteren Grenzflächenbereich des entstehenden Gusskörpers erfolgt. Dieser untere Grenzflächenbereich kann sodann zur Bildung des Berührungsbereichs 2" herangezogen werden.

Im Sinne einer ausreichend hohen mechanischen Stabilität ist in sämtlichen vorgenannten Ausführungsbeispielen für das Matrixmaterial, das in der Art eines Bindemittels die mechanische Stabilität unter den Kupfer-Partikeln 10 herstellt, ein geeignet gewählter Volumenanteil an der Oberflächenschicht 6 insgesamt vorgesehen. Um dem Rechnung zu tragen, beträgt im Ausführungsbeispiel der Volumenanteil der Kupfer-Partikel 10 am Gesamtvolumen der Oberflächenschicht 6 zwischen 5% und 40%.

Die Herstellung des Desinfektionselements 1 ist für alle Varianten, abgesehen von der Bereitstellung der frei zugänglichen Oberfläche der Kupfer-Partikel 10, und abgesehen von dem Umstand, ob ein Vollkörper 20 oder eine Beschichtung 14 gewünscht ist, mehr oder weniger identisch. Nachfolgend werden daher einige Beispiele zur Herstellung der Beschichtung 14 näher erläutert, die sich hinsichtlich der erzielten Ergebnisse - ggf. unter Rückgriff auf geeignete standardisierte Gussverfahren - problemlos auch auf die Herstellung von Vollkörpern 20 übertragen lassen. Insbesondere hat sich für die Herstellung der Beschichtung 14, unter Anwendung eines Tauchbeschichtungsverfahrens, ein Vorgehen entsprechend der nachfolgend aufgeführten Beispiele als besonders vorteilhaft erwiesen:

### 1. Vorbereitung des Tragkörpers 12:

Als Tragkörper 12 wurden versuchsweise ein Stahl-Substrat ("Low Carbon Steel"), ein Polyethylen-Substrat und ein Glas-Substrat beschichtet.

Vor der Beschichtung wurde eine Aufrauung vorgenommen, um die Anhaftung der Beschichtung 14 zu verbessern. Anschließend wurden die Substrate mit Isopropanol und destilliertem Wasser gereinigt und einer Trockung über einen Zeitraum von 24 h unterzogen.

### 2. Stabilisierung der Kupfer-Partikel 10:

Zur Stabilisierung wurden die Kupfer-Partikel 10 zunächst einem Behandlungsschritt mit Ultraschall in Ethanol für 4 Min. unterzogen. Anschließend wurde Polyvinylpyrrolidon (PVP) zur Mischung hinzugegeben und erneut eine Ultraschallbehandlung über einen Zeitraum von etwa 4 Min. vorgenommen. Anschließend erfolgte eine Trocknung bei einer Temperatur von 90° C über einen Zeitraum von 24h, wobei das Lösungsmittel vollständig verdampft wurde. Durch diese Behandlung mit Polyvinylpyrrolidon (PVP), eine so genannte sterische Stabilisierung, wird die Oberfläche der Kupfer-Partikel 10 funktionalisiert, so dass nachfolgend ein Verklumpen oder eine Agglomeration verhindert wird.

### 3. Vermischung mit dem Matrixbildner und Aufbringung der Beschichtung 14:

Die stabilisierten Kupfer-Partikel 10 wurden mit dem Matrixbildner vermischt und anschließend durch Tauchbeschichtung auf den Tragkörper 12 aufgebracht. Folgende Alternativen wurden getestet und als vorteilhaft erkannt:

### - Matrixbildner Polyparaphynelen (PPP):

Der Matrixbildner PPP (beispielsweise erhältlich als "PrimoSpire PR-250" des Herstellers Solvay Specialty Polymers) wurde in der benötigten Menge in Chloroform gelöst. Nach der Vermischung wurde das Gemisch zunächst für 60 min. bei moderaten Drehzahlen (200 U/min) gerührt, um eine vollständige Lösung sicherzustellen. Anschließend wurde die erforderliche Menge an stabilisierten Kupfer-Partikeln 10 beigemischt. Zur Homogenisierung wurde diese Mischung bei hohen Drehzahlen (6.500 U/min bis 9.500 U/min) für einen Zeitraum von einigen Minuten gerührt. Anschließend wurde der Tragkörper 12 zum Zweck der Tauchbeschichtung in die homogenisierte Lösung eingetaucht und anschließend für 24 h getrocknet. Um evtl. noch verbliebene Chloroform-Rückstände zu verdampfen, erfolgte sodann eine Wärmebehandlung bei 90°C im Vakuum für etwa 48 h.

### - Matrixbildner Epoxyharz:

Die erforderliche Menge an stabilisierten Kupfer-Partikeln 10 wurde dem Matrixbildner (beispielsweise erhältlich als "EPON 28") beigemischt. Zur Homogenisierung wurde diese Mischung bei hohen Drehzahlen (6.500 U/min bis 9.500 U/min) für einen Zeitraum von einigen Minuten gerührt und anschließend abkühlen gelassen. Die erforderliche Menge an Harzbildner (beispielsweise EPIKUR 3223) wurde in einem Verhältnis Harz/Harzbildner = 10:1 beigemischt, und das Gemisch wurde von Hand gerührt. Anschließend wurde der Tragkörper 12 zum Zweck der Tauchbeschichtung in die homogenisierte Lösung eingetaucht und anschließend für 24 h getrocknet. Die Harzbildung wurde durch eine nachfolgende Wärmebehandlung bei 100°C für etwa 4 h abgeschlossen.

### - Matrixbildner Polyurethan (PU):

Der Matrixbildner PU (beispielsweise erhältlich als "TECOFLEX RESIN EG65D" des Herstellers Lubrizol) wurde in der benötigten Menge Lösungsmittel (beispielsweise n,n-dimethylformamid, DMF, erhältlich als ANHYDROUS 99,8% des Herstellers Sigma Aldrich) gelöst und einer Ultraschallbehandlung über 3 h unterzogen, um die vollständige Auflösung des Matrixbildners im Lösungsmittel sicherzustellen. Anschließend wurde die erforderliche Menge an stabilisierten Kupfer-Partikeln 10 beigemischt. Zur Homogenisierung wurde diese Mischung bei hohen Drehzahlen (6.500 U/min bis 9.500 U/min) für einen Zeitraum von einigen Minuten gerührt. Anschließend wurde der Tragkörper 12 zum Zweck der Tauchbeschichtung in die homogenisierte Lösung eingetaucht und anschließend für 24 h getrocknet. Um evtl. noch verbliebene Rückstände an Lösungsmitteln zu verdampfen, erfolgte sodann eine Wärmebehandlung bei 60°C im Vakuum für etwa 48 h.

### 4. Ergebnisse:

Die antibakterielle Wirkung wurde mittels eines so genannten "wet plating" Verfahrens [Wilks et al., 2005, Int. J. Food Microbiol. 105:445-454] an E. coli K12 (ATCC 23716) getestet. Dazu wurde die Bakterienkultur über 24 h bei 37°C in Luria-Bertani Medium bis zu einer optischen Dichte von 2,1 bis 2,3 herangezogen. 10 ml dieser Bakteriensuspension mit einer Konzentration von etwa 109 pro ml wurden bei 4000 rpm für 10 min zentrifugiert. Die zentrifugierten Bakterien wurden im Anschluss mit 10 ml 0,9% NaCl-Lösung durch Vortexen vermischt und 1:10 verdünnt.

Zum Test der antibakteriellen Eigenschaften wurden 25 µl der entsprechende Bakteriensuspension mit festgelegter Konzentration von etwa 10⁸ Bakterien auf die Probe gegeben und nach festgelegten Kontaktzeiten von 60 min, 120 min, 180 min und 240 min wieder von der Probe mittels Pipette entfernt. Die jeweiligen Verdünnungen der entfernten Proben wurden auf LB-Agarplatten ausplattiert und die koloniebildenden Einheiten nach 24 h ausgezählt. Als Referenz wurde bei allen Proben eine Polycarbonatplatte verwendet, welche keine mikrobiziden Eigenschaften aufweist.

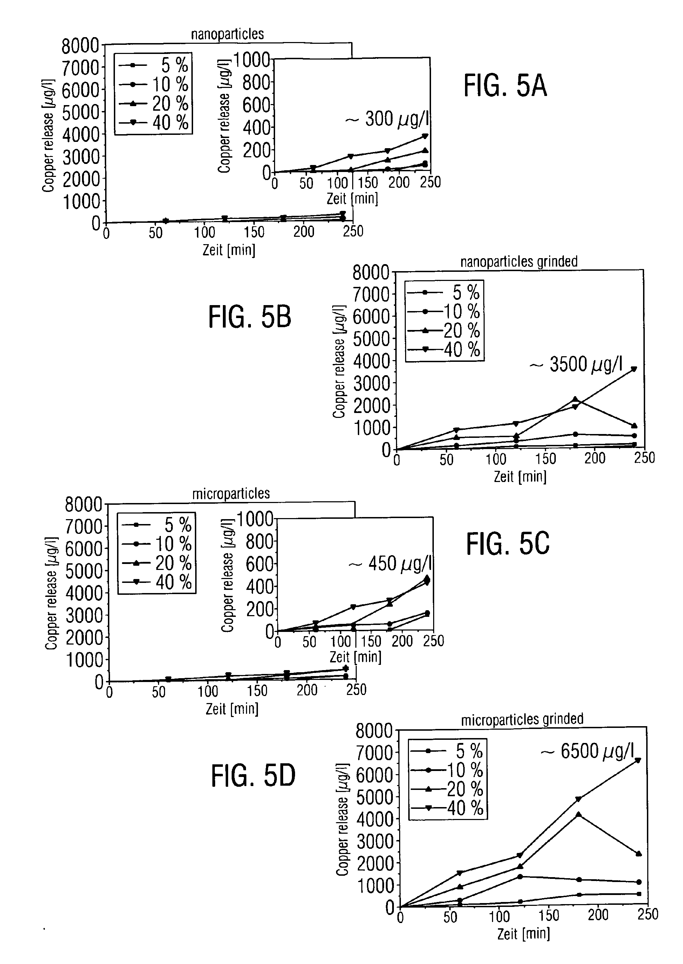

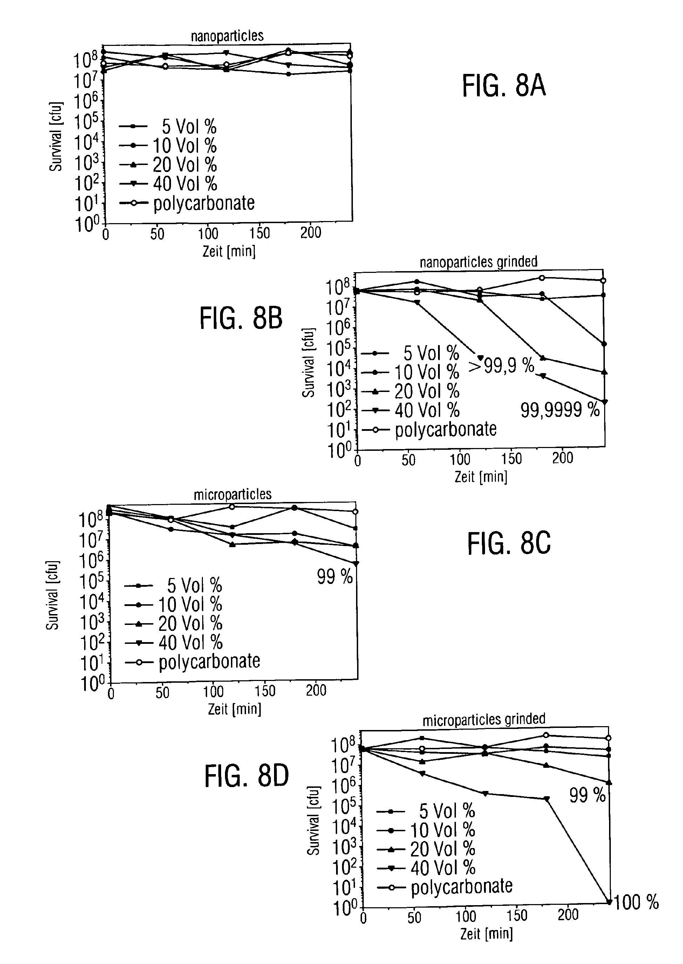

Die Kupferionenabgabe wurde mittels Atomabsorptionsspektrometrie an denselben Proben gemessen, um neben der antibakteriellen Wirksamkeit die abgegebene Kupferkonzentration in den Bakteriensuspensionen zu messen. Diese lässt sich unter Umständen zur Quantifizierung antibakterieller Eigenschaften kupferbasierter Systeme verwenden [Molteni et al., 2010, Appl. Environ. Microbiol., vol. 76 no. 12 4099-4101]. Die Ergebnisse der Messungen zur Kupferionenabgabe sind in den FIG. 5-7 und die Ergebnisse zur mikrobiziden Wirkung in den FIG. 8-10 dargestellt.

In den Diagrammen gem. FIG. 5 ist die Kupferabgabe (oder "Ionenauschüttung") in Abhängigkeit von der Zeit bei verschiedenen Konzentrationen an Kupfernanopartikeln ungeschliffen (FIG. 5a), Kupfernanopartikeln geschliffen (FIG. 5b), Kupfermikropartikeln ungeschliffen (FIG. 5c) und Kupfermikropartikeln geschliffen (FIG. 5d) dargestellt. Mit "unge-schliffen" bzw. "geschliffen" ist dabei bezeichnet, ob die durch die Matrix 8, in die die Kupfer-Partikel 10 eingebettet sind, gebildete Beschichtung 14 nach dem Aushärten des Matrixbildners einer Oberflächenbehandlung (Aufrauhen) durch mechanisches Schleifen unterzogen wurde. Die Partikel sind dabei in Polyparaphenylen eingebettet. Die Messung wurde mittels Atomabsorptionsspektrometrie durchgeführt.

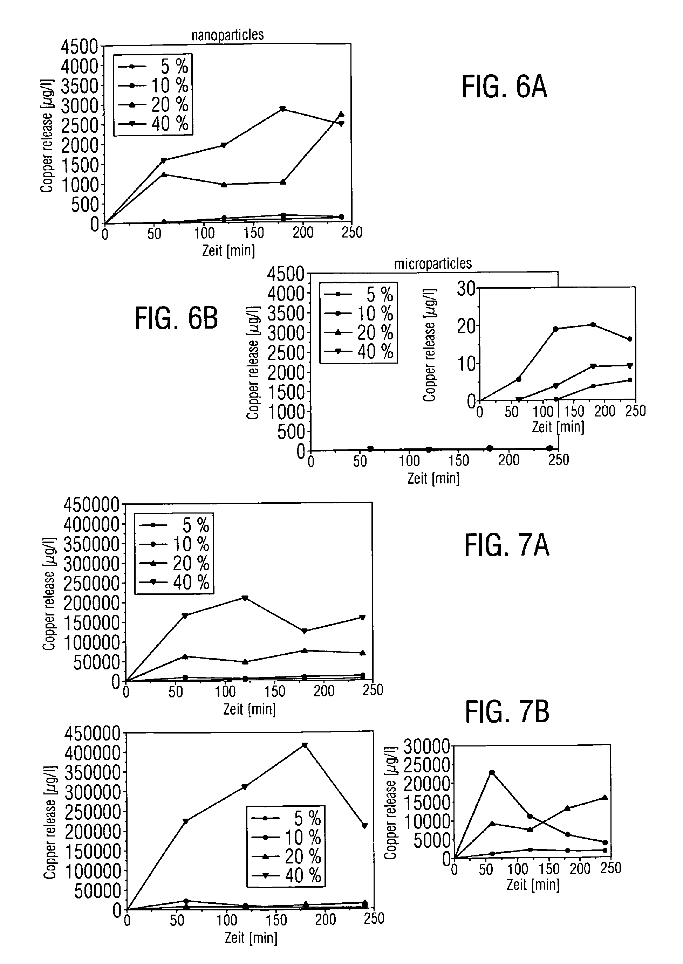

Im Vergleich zeigen die Diagramme gem. FIG. 6 die Kupferabgabe in Abhängigkeit der Zeit bei verschiedenen Konzentrationen an Kupfernanopartikeln (FIG. 6a) und Kupfermikropartikeln (FIG. b), eingebettet in Polyurethan. Die Messung wurde mittels Atomabsorptionsspektrometrie durchgeführt.

In FIG. 7 hingegen ist in Form der Diagramme die Kupferabgabe in Abhängigkeit der Zeit bei verschiedenen Konzentrationen an Kupfernanopartikeln (FIG. 7a) und Kupfermikropartikeln (FIG. 7b), eingebettet in Epoxidharz, dargestellt. Die Messung wurde ebenfalls mittels Atomabsorptionsspektrometrie durchgeführt.

Die Ergebnisse der Messungen zur Kupferionenabgabe verschiedener Polymer-Kupfer-Komposite zeigen durchweg einen Anstieg der Kupferkonzentration in Abhängigkeit der Zeit, mit Ausnahme der Epoxidharz-Systeme, welche nach 120 min ihren Sättigungswert erreichen. Desweiteren beobachtet man, dass mit steigender Konzentration an Kupfernanopartikeln sowie -mikropartikeln auch die Kupferionenabgabe ansteigt. Dabei lassen sich signifikante Werte bei 5 Vol% nicht erkennen.

Bei Polyparaphenylen zeigen die Messungen, dass Mikropartikel (bei 40 Vol% etwa 400 µg/l nach 240 min) im Vergleich zu Nanopartikeln (bei 40 Vol% etwa 300 µg/l nach 240 min) eine ähnliche bis leicht größere Ionenabgabe hervorrufen. Desweitern lässt sich ein enormer Anstieg der Kupferkonzentration beobachten, wenn beide Proben nach der Herstellung angeschliffen werden. Dieser Effekt quantifiziert sich in einer Steigerung um mehr als eine Größenordnung.

Polyurethan in Kombination mit Mikropartikeln hingegen lässt keinen signifikanten Effekt erkennen (unter 20 µg/l für alle Konzentrationen), wobei das Nanopartikel-Komposit eine ähnliche Ionenabgabe hervorruft wie die geschliffene Variante aus Polypraphenylen.

Die Ionenabgabe bei Epoxidharz ist im Vergleich zu allen anderen Systemen um etwa zwei Größenordnungen höher. Dabei ist der Effekt bei Mikropartikeln (bei 40 Vol% etwa 400 mg/l nach 180 min) um den Faktor 2 größer als bei Nanopartikeln.

Es lässt sich somit festhalten, dass die Polymersysteme aus Polyparaphenylen und Epoxidharz zusammen mit Mikropartikeln eine höhere Ionenabgabe aufweisen als dieselben mit Nanopartikeln und dass eine Vorbehandlung von Polyparaphenylen durch mechanisches Schleifen die Kupferionenabgabe um ein Vielfaches steigern kann.

Zur Verdeutlichung der mikrobiziden Eigenschaften des hergestellten Komposits ist in den Diagrammen gem. FIG. 8 die Anzahl koloniebildender Einheiten (überlebende Bakterien; hier: E.coli) in Abhängigkeit der Zeit bei verschiedenen Konzentrationen an Kupfernanopartikeln ungeschliffen (FIG. 8a), Kupfernanopartikeln geschliffen (FIG. 8b), Kupfermikropartikeln ungeschliffen (FIG. 8c) und Kupfermikropartikeln geschliffen (FIG. 8d) gezeigt, wobei die Partikel in Polyparaphenylen eingebettet waren. Die Messung wurde mittels der "wet plating"-Methode durchgeführt.

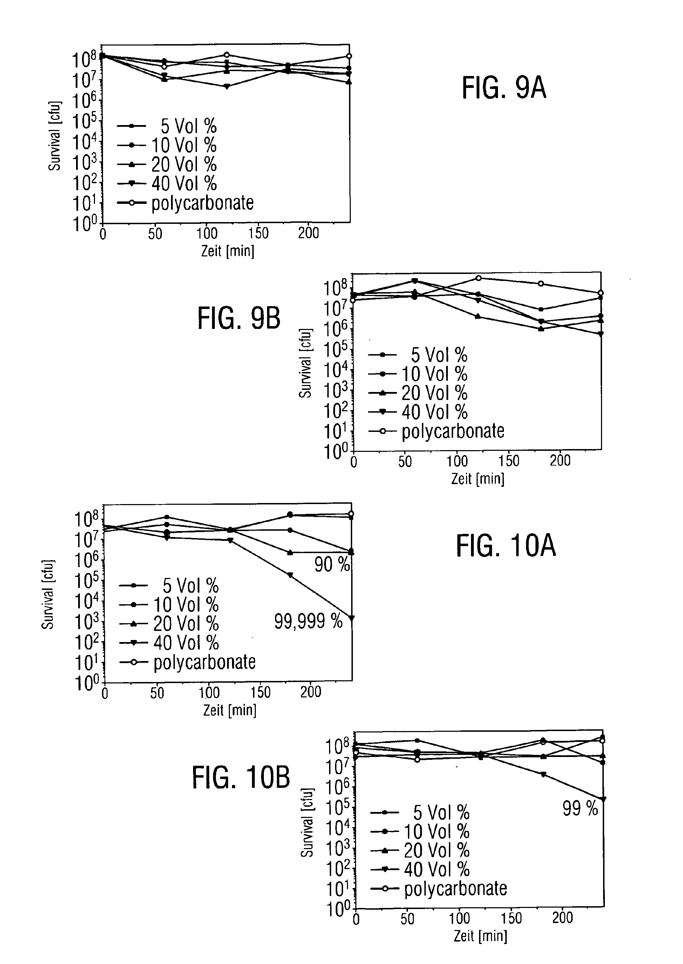

Die Diagramme in FIG. 9 zeigen die Anzahl koloniebildender Einheiten (überlebende Bakterien; hier: E.coli) in Abhängigkeit der Zeit bei verschiedenen Konzentrationen an Kupfernanopartikeln (FIG. 9a) und Kupfermikropartikeln (FIG. 9b), wobei die Partikel in Polyurethan eingebettet waren. Auch diese Messung wurde mittels der "wet plating"-Methode durchgeführt. In den Diagrammen gem. FIG. 10 ist zudem die Anzahl koloniebildender Einheiten (überlebende Bakterien; hier: E.coli) in Abhängigkeit der Zeit bei verschiedenen Konzentrationen an Kupfernanopartikeln (FIG. 10a) und Kupfermikropartikeln (FIG. 10b), wobei die Partikel in Epoxidharz eingebettet waren. Diese Messung wurde ebenfalls mittels der "wet plating"-Methode durchgeführt.

Die Ergebnisse zur antibakteriellen Wirkung gegen E.coli zeigen, dass Polyparaphenylen und Polyurethan als Matrixbildner sowohl mit Nanopartikeln als auch mit Mikropartikeln ohne weitere Behandlung keine signifikante mikrobizide Wirkung zeigen. Die geschliffenen Proben aus Polyparaphenylen zeigen jedoch einen erheblichen mikrobiziden Effekt, welcher sich in kompletter Tötung bei 40 Vol% Mikropartikeln nach 240 min äußert. Durch die Verwendung von Nanopartikeln mit einer Konzentration von 40 Vol% lassen sich nach 240 min noch mehr als 100 Bakterien nachweisen.

Die Epoxidharz-Komposite zeigen im Vergleich zu Polyparaphenylen auch ohne mechanische Vorbehandlung einen signifikanten Effekt. Die Grafiken in FIG. 10 zeigen, dass bei 40 Vol% und der Verwendung von Nanopartikeln nur noch etwa 1000 Bakterien nachzuweisen waren. Die Verwendung von Mikropartikeln mit sonst gleichen Parametern induziert lediglich eine Reduktion der Bakterien um mehr als zwei log-Stufen.

Demnach kann man abschließend sagen, dass mechanisches Schleifen der Polymeroberfläche (am Beispiel von Polyparaphenylen) die antibakterielle Wirkung enorm steigern kann und dass die Verwendung von Mikropartikeln dabei eine wesentlich höhere Wirkung hat als die Verwendung von Nanopartikeln. Außerdem konnte gezeigt werden, dass Epoxidharze auch ohne mechanische Nachbehandlung eine starke antibakterielle Wirkung aufweisen, wobei Nanopartikel eine geringfügig schnellere Wirkung haben als Mikropartikel.

### Bezugszeichenliste

- 1,1': Desinfektionselement
- 2,2': Berührungsbereich
- 4: Oberfläche
- 6: Oberflächenschicht
- 8: Matrix
- 10: Kupfer-Partikel
- 12: Tragkörper
- 14: Beschichtung
- 20: Vollkörper

## Patentansprüche

1. Desinfektionselement (1,1') mit einem Berührungsbereich (2,2'), der eine mikrobizide, aus in eine Matrix (8) eingebetteten Kupfer-Partikeln (10) gebildete Oberflächenschicht (6) aufweist.

2. Desinfektionslement (1,1') nach Anspruch 1, bei dem die mikrobizide Oberflächenschicht (6) als auf einen Tragkörper (12) aufgebrachte Beschichtung (14) ausgeführt ist.

3. Desinfektionselement (1,1') nach Anspruch 1 oder 2, bei dem die in die Matrix (8) eingebetteten Kupfer-Partikel (10) eine Partikelfraktion mit einer Partikelgröße von höchstens 500 nm, vorzugsweise mit einer Partikelgröße von 90 bis 250 nm, umfassen.

4. Desinfektionselement (1,1') nach einem der Ansprüche 1 bis 3, bei dem die in die Matrix (8) eingebetteten Kupfer-Partikel (10) eine Partikelfraktion mit einer Partikelgröße von mindestens 1 µm umfassen.

5. Desinfektionselement (1,1') nach Anspruch 4, bei dem die Partikelfraktion eine mitt-lere Partikelgröße von etwa 50 µm mit einer Standardabweichung von höchstens 20% aufweist.

6. Desinfektionselement (1,1') nach einem der vorhergehenden Ansprüche, bei dem die Matrix (8) aus Polymermaterial, vorzugsweise aus Paraphynelen, aus einem Epoxyharz und/oder aus Polyurethan, gebildet ist.

7. Desinfektionselement (1,1') nach einem der vorhergehenden Ansprüche, bei dem die Kupfer-Partikel (10) im Oberflächenbereich der Schicht (6) zumindest teilweise
unbedeckt vom Matrixmaterial vorliegen.

8. Desinfektionselement (1,1') nach Anspruch 7, bei dem der durch die frei liegenden Kupfer-Partikel (10) gebildete Anteil der Gesamtoberfläche der Oberflächenschicht (6) mindestens 10 % beträgt.

9. Desinfektionselement (1,1') nach einem der vorhergehenden Ansprüche, bei dem der Volumenanteil der Kupfer-Partikel (10) am Gesamtvolumen der Oberflächenschicht (6) zwischen 10% und 80% beträgt.

10. Desinfektionselement (1,1') nach einem der vorhergehenden Ansprüche, erhältlich durch Abscheidung einer mit Kupfer-Partikeln (10) und einem Matrixbildner versehenen Beschichtungslösung auf einem Trägerkörper (12), vorzugsweise durch Tauchbeschichtung, zur Bildung des Berührungsbereichs (2,2').

11. Verfahren zur Herstellung eines Desinfektionselements (1,1') nach einem der vorhergehenden Ansprüche, bei dem ein Trägerkörper (12) zumindest teilweise mit einer mit Kupfer-Partikeln (10) und einem Matrixbildner versehenen Beschichtungslösung beschichtet wird.

12. Verfahren nach Anspruch 11, bei dem die Beschichtungslösung durch Einrühren der Kupfer-Partikel (10) in eine Lösung des Matrixbildners und anschließendes Homogenisieren bereitgestellt wird.

13. Verfahren nach Anspruch 12, bei dem die Kupfer-Partikel (10) vor dem Einbringen in die Lösung des Matrixbildners stabilisiert werden.

14. Verfahren nach Anspruch 13, bei dem die Kupfer-Partikel (10) zur Stabilisierung und Homogenisierung einem Behandlungsschritt unterzogen werden, bei dem sie in einer Mischung aus einem Lösungsmittel, vorzugsweise Ethanol, und Polyvinylpyrrolidon (PVP) mit Ultraschall beaufschlagt werden.
